# NEUE EUROPÄISCHE PATENTSCHRIFT

(11) **EP 1 463 710 B2**
(45) Veröffentlichungstag und Bekanntmachung des Hinweises auf die Entscheidung über den Einspruch: **15.02.2012**
(45) Hinweis auf die Patenterteilung: 25.06.2008
(21) Anmeldenummer: 02769989.1
(22) Anmeldetag: 03.09.2002
(51) Int. Cl.: C07C 67/303, C07C 69/75, C08K 5/12

(54) **VERFAHREN ZUR HERSTELLUNG VON ALICYCLISCHEN POLYCARBONSÄUREESTERGEMISCHEN MIT HOHEM TRANS-ANTEIL**
METHOD FOR THE PRODUCTION OF ALICYCLIC POLYCARBOXYLIC ACID ESTER MIXTURES WITH A HIGH TRANS PROPORTION
PROCEDE DE FABRICATION DE MELANGES D'ESTERS D'ACIDES POLYCARBOXYLIQUES ALICYCLIQUES A FRACTION TRANS ELEVEE

(30) Priorität: 24.09.2001 DE 10146869
(43) Veröffentlichungstag der Anmeldung: 06.10.2004
(73) Patentinhaber: Evonik Oxeno GmbH, 45772 Marl (DE)
(72) Erfinder: MASCHMEYER, Dietrich, 45657 Recklinghausen (DE); BÜSCHKEN, Wilfried, 45721 Haltern (DE); GRASS, Michael, 45721 Haltern (DE); TUCHLENSKI, Axel, 45478 Mülheim (DE)
(74) Vertreter: Hirsch, Hans-Ludwig
(86) Internationale Anmeldenummer: PCT/EP2002/009805
(87) Internationale Veröffentlichungsnummer: WO 2003/029181

(56) Entgegenhaltungen:
- EP-A- 0 005 737
- DE-A- 19 927 978
- JP-A- 2001 207 002
- US-A- 3 027 398
- US-A- 3 326 972
- US-A- 3 428 668

## Beschreibung

Die vorliegende Erfindung betrifft ein Verfahren zur Herstellung alicyclischer Polycarbonsäureester mit hohem trans-Anteil durch Kernhydrierung der entsprechenden aromatischen Polycarbonsäureester.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine wie PVC Verwendung.

Für die Weichmachung von PVC werden zur Zeit überwiegend Ester der Phthalsäure, wie beispielweise Dibutyl- , Dioctyl-, Dinonyl- oder Didecylester, verwendet. Da diese Phthalate in letzter Zeit als gesundheitsschädlich bezeichnet werden, muss befürchtet werden, dass deren Einsatz in Kunststoffen eingeschränkt werden könnte. Alicyclische Polycarbonsäurester, von denen einige in der Literatur als Weichmacher für verschiedene Kunststoffe beschrieben sind, könnten dann als Ersatzstoffe, wenn auch mit einem etwas anderem anwendungstechnischen Profil, zur Verfügung stehen.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:

In US 5 286 898 und US 5 319 129 wird ein Verfahren beschrieben, mit dem Dimethylterephthalat an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250°C und 30 bis 200 bar hydriert. US 3 027 398 offenbart die Hydrierung von Dimethylterephthalat an geträgerten Ru-Katalysatoren bei 110 bis 140°C und 35 bis 105 bar.

In WO 00/78704 wird ein Verfahren zur Hydrierung von Benzolpolycarbonsäureester zu den entsprechenden alicyclischen Verbindungen offengelegt. Dabei werden Trägerkatalysatoren eingesetzt, die Ru alleine oder zusammen mit mindestens einem Metall der I., VII. oder VIII. Nebengruppe des Periodensystems enthalten und 50% Makroporen aufweisen.

Bei der Kernhydrierung von aromatischen Polycarbonsäureestern können bezüglich des Ringsystems und der Esterfunktionen mindestens zwei Isomere entstehen.

Beispielsweise sind bei der Hydrierung von Phthalsäurediester (1,2-Benzoldicarbonsäureester) cis- und/oder trans-Cyclohexan-1,2-dicarbonsäurediester die Produkte. Dabei versteht man unter cis-Diester dasjenige Isomer, bei dem die eine Estergruppe axial (a) und die andere äquatorial (e) ausgerichtet ist. Unter trans-Verbindung ist dasjenige Isosomer zu verstehen, bei dem beide Estergruppen entweder axial (a, a) oder äquatorial (e, e) ausgerichtet sind.

Bei der Hydrierung von Isophthalsäurediester (1,3-Benzoldicarbonsäurediester) können cis- und trans-1,3-Cyclohexancarbonsäurediester entstehen. Bei der cis-Verbindung sind die Estergruppen entweder axial-axial (a, a) oder äquatorial-äquatorial (e, e) ausgerichtet. Bei der trans Verbindung ist eine Estergruppe axial und die andere äquatorial ausgerichtet.

Bei der Hydrierung von Terephthalsäurediester (1,4-Benzoldicarbonsäurediester) können cis- und trans-1,4-Cyclohexandicarbonsäurediester entstehen. Dabei sind bei der cis-Verbindung eine Estergruppe axial und die andere äquatorial (a, e) ausgerichtet. Bei der trans-Verbindung sind beide Estergruppen entweder axial (a, a) oder äquatorial (e, e) ausgerichtet.

Bei alicyclischen Polycarbonsäureester mit mehr als zwei Substituenten am gleichen Ringsystem kann jeder Substituent zu einem anderen Substituenten cis- oder trans-konfiguriert sein. Im Sinne der vorliegenden Erfindung sind alle Verbindungen, bei denen die Mehrzahl der Estergruppen trans zueinander konfiguriert sind, unabhängig von den anderen Konfigurationen der Substituenten zueinander als trans-Verbindungen anzusehen.

Über die Konfiguration der Produkte, die bei der Kernhydrierung von aromatischen Polycarbonsäureestern entstehen, findet man in der Literatur nur wenige und lückenhafte Informationen.

Beispielweise entsteht nach US 3 027 165 bei der Hydrierung von Dimethyltherephthalat an einem Rutheniumkatalysator ein Gemisch aus cis- und trans-1,4-Cyclohexandicarbonsäuredimethylester mit einem Schmelzpunkt von unter 20 °C. Es ist bekannt, dass der Schmelzpunkt des trans-Diesters 70 °C und der des cis-Diesters 7 °C beträgt. Bei Annahme des üblichen Schmelzverhalten (keine Mischkristallbildung), d. h., dass ausgehend von einem reinen Isomeren bei Zugabe des anderen Isomeren der Schmelzpunkt des Gemisches so lange sinkt, bis der eutektische Punkt erreicht ist, kann abgeschätzt werden, dass das Hydriergemisch hauptsächlich aus cis-1,4-Cyclohexandicarbonsäuredimethylester besteht.
S. Siegel und G. McCaleb in JACS, 81, 1959, S. 3655-3658 beschreiben die Hydrierung von Phthalsäuredimethylestem an suspendiertem Platinoxidpulver in Eisessig. Es wurden druck- und konzentrationsunabhängig Gemische der entsprechenden Cyclohexansäurederivate mit einem cis-Anteil von praktisch 100 Mol-% erhalten.
Die Herstellung von Cyclohexandicarbonsäuredimethylester mit einem hohen cis-Anteil ist daher bekannt. Es ist dieser Publikation jedoch nicht zu entnehmen, ob auch andere Carbonsäureester mit hohem cis-Anteil durch die publizierte oder eine andere Methode zugänglich sind.

Werden die in WO 00/78704 offenbarten Ruthenium-haltigen Katalysatoren zur Hydrierung von Diisononylphthalaten eingesetzt, wird ein Produktgemisch mit ca. 93 Mol-% cis- und entsprechend 7 Mol-% des trans-Isomers erhalten.
EP 0 005 737 beschreibt eine Hydrierung von aromatischen Carbonsäureestern an einem Festbettkatalysator, der etwa auch Nickel enthalten kann. Zum cis-/trans-Verhältnis des Reaktionsprodukts werden keine Aussagen gemacht.
US 3 428 668 betrifft die Herstellung von 1,4-Cyclohexandicarbonsäuredialkylester an einem Nickel-Katalysator. Angaben zum cis-/trans-Verhältnis können nicht entnommen werden. Katalysatoren, die Nickel und Zink aufweisen, werden dabei nicht eingesetzt.
Gezielt hergestellte alicyclische Polycarbonsäureestergemische mit einem erhöhten Anteil der trans-Isomeren sind in der einschlägigen Literatur nicht belegt.
Aufgabe der vorliegenden Erfindung war es daher, solche Gemische herzustellen und auf ihre Verwendung als Weichmacher zu prüfen.
Aufgabe der vorliegenden Erfindung war es weiterhin, ein Verfahren zur Hydrierung von aromatischen Polycarbonsäuren- Estern zu entwickeln, mit dem ohne Edelmetallkatalysatoren Cyclohexandicarbonsäureverbindungen mit guten Selektivitäten und Ausbeuten hergestellt werden können.

Gegenstand der Erfindung ist daher ein Verfahren zur katalytischen Hydrierung von aromatischen Polycarbonsäuren Derivaten mit Wasserstoff gemäß Anspruch 1, wobei insbesondere die Hydrierung an einem Katalysator, der mindestens Nickel zusammen mit Zink sowie zusätzlich eine inerte Komponente enthält, durchgeführt wird, wobei als aromatische Polycarbonsäure oder deren Derivate die Alkyl-, Cycloalkyl- oder Alkoxyalkylester der 1,2-Benzoldicarbonsäure eingesetzt wird.

Die erfindungsgemäß hergestellten alicyclischen Polycarbonsäureester (trans-Anteil > 10 %) zeichnen sich gegenüber den entsprechenden Gemischen mit einem kleineren trans-Anteil durch tendenziell geringere Flüchtigkeit aus. Dies ist insbesondere dann von Bedeutung, wenn hieraus Kunststoffartikel für Innenraumanwendungen gefertigt werden sollen.

Die geringere Flüchtigkeit dieser Gemische, die sich beispielsweise über dynamische Thermogravimetrie (TGA) belegen läßt, führt auch bei Kunststoffartikeln zu geringeren Masseverlusten nach thermischer Alterung als bei der Verwendung von Estergemischen mit geringeren trans-Gehalten als Weichmacher. Somit sind solche Gemische den herkömmlichen Polycarbonsäureestergemischen aus anwendungstechnischer Sicht überlegen.
Die Herstellung der Gemische erfolgt durch Hydrierung der entsprechenden aromatischen Polycarbonsäureester. Hierzu können insbesondere die im Folgenden genannten Katalysatorsysteme eingesetzt werden.
Bevorzugte Metalle der II, III, IV, V, und/oder VI Nebengruppe sind Zink und/oder Chrom .

Darüber hinaus können alle im erfindungsgemäßen Verfahren eingesetzten Katalysatoren zusätzlich eine inerte Komponente (Träger), die mindestens ein Metall aus der Gruppe Al, Mg, Ti, Zr und/oder Si, als Oxid oder Mischoxid enthält, aufweisen. Optional können die Katalysatoren auch Salze der oben genannten Metalle, wie beispielsweise Sulfate und/oder Phosphate enthalten. Weiterhin können die erfindungsgemäß eingesetzten Katalysatoren Verarbeitungs- und Fornigebungshilfsmittel wie beispielsweise Graphit beinhalten.

Die im Folgenden angegebenen Zusammensetzungen beziehen sich auf die reduzierten Katalysatoren.

Der Gehalt der Katalysatoren an den genannten Metallen der VIII Nebengruppe (berechnet als Metall) liegt im Bereich von 1 bis 60 Massen-%, insbesondere im Bereich von 25 bis 45 Massen. %, ganz besonders zwischen 30 und 40 Massen-%.

Der Gehalt der Katalysatoren an Metallen der II, III, IV, V und/oder VI Nebengruppe (berechnet als Oxid) beträgt 10 bis 90 Massen-%, insbesondere 20 bis 60 Massen-%, ganz besonders 20 bis 40 Massen-%.

Im erfindungsgemäßen Verfahren werden besonders bevorzugt Katalysatoren eingesetzt, die in reduzierter, aktiver Form Nickel zumindest teilweise in der Oxidationsstufe 0 und Zink vorzugsweise in der Oxidationsstufe +2 enthalten.

Die Katalysatoren werden nach an sich bekannten Verfahren hergestellt. Um einen Katalysator herzustellen, der die Hauptkomponenten Nickel, Zinkoxid und als Träger Siliciumdioxid enthält, werden beispielsweise Nickel- und Zinkcarbonat in einer Suspension von Kieselsäure und gegebenenfalls Graphit in Wasser ausgefällt. Weitere, dem Fachmann bekannte Schritte zur Herstellung des Katalysators sind: Abtrennung und Wäsche des Niederschlags, Trocknung, Calcinierung, Formgebung und Reduktion.

Die Katalysatoren werden zweckmäßig in eine Form gebracht, die bei der Hydrierung einen geringen Strömungswiderstand bietet, wie beispielsweise Tabletten, Zylinder, Strangextrudate oder Ringe.

Im erfindungsgemäßen Verfahren kann beispielsweise der käuflich verfügbare Katalysator H10126 der Degussa AG, Düsseldorf, eingesetzt werden. Dieser Katalysator wird bislang nur für die Hydrierung von aromatischen und olefinischen Kohlenwasserstoffen in Halogen- und Schwefel enthaltenden Rohstoffen eingesetzt. Sein Einsatz für die Kernhydrierung von aromatischen Estern ist nicht bekannt. Dieser Katalysator enthält 32 Massen-% Nickel, 29 Massen-% Zinkoxid, 24 Massen-% Siliciumdioxid.

Im erfindungsgemäßen Verfahren wird die Hydrierung bevorzugt in flüssiger Phase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt/Eduktphase hauptsächlich im flüssigen Zustand befindet, bevorzugt.

Wenn die Hydrierung kontinuierlich an einem im Festbett angeordneten Katalysator durchgeführt wird, ist es zweckmäßig, den Katalysator vor der Hydrierung in die aktive Form überzuführen. Dies kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden.

Für das erfindungsgemäße Verfahren können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Polycarbonsäureester im geraden Durchgang oder unter Produktrückführung zu hydrieren.

Das erfindungsgemäße Verfahren wird in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-ZeitAusbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

### Beispielsweise können folgende Stoffe als Lösemittel eingesetzt werden:

Geradkettige oder cyclische Ether, wie beispielsweise Tetrahydrofuran oder Dioxan, sowie aliphatische Alkohole, in denen der Alkylrest 1 bis 13 Kohlenstoffatome aufweist.
Bevorzugt verwendbare Alkohole sind beispielsweise Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde (z. B. Isononanol bei der Hydrierung von Diisononylphthalaten). Dadurch ist die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zu Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch die Zirkulationsrate (Mengenverhältnis von rückgeführten Hydrieraustrag zu Edukt) eingestellt werden.

Das erfindungsgemäße Verfahren wird in einem Druckbereich von 30 bis 250 bar, insbesondere zwischen 50 und 100 bar durchgeführt. Die Hydriertemperaturen liegen zwischen 80 und 200, insbesondere zwischen 100 und 140 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Inertgasbestandteile können beispielsweise Stickstoff oder Methan sein. Bevorzugt wird Wasserstoff in einer Reinheit von größer 95 %, insbesondere größer 98 % eingesetzt.

Nach dem erfindungsgemäßen Verfahren können aromatische Polycarbonsäuren Alkylester zu den entsprechenden alicyclischen Polycarbonsäureverbindungen umgesetzt werden.

Die nach dem erfindungsgemäßen Verfahren hergestellten Polycarbonsäureester enthalten, 2, Estergruppen.

Die im erfindungsgemäßen Verfahren verwendeten Polycarbonsäureester sind Benzol-, polycarbonsäureester. Die so erhaltenen alicyclischen Polycarbonsäureester bestehen aus einem C₆-Ring.

Die Alkoholkomponente der Polycarbonsäureester besteht bevorzugt aus verzweigten oder unverzweigten Alkyl-, Cycloalkyl- oder Alkoxyalkylgruppen mit 8-13 Kohlenstoffatomen. Diese können in einem Molekül eines Polycarbonsäureesters gleich oder unterschiedlich sein, d. h. sie können gleiche oder verschiedene Isomeren oder Anzahl Kohlenstoffatome besitzen.

In der Ausführungsform betrifft die vorliegende Erfindung ein Verfahren zur Hydrierung der 1,2-; Benzoldicarbonsäureester, d. h. die Gemische enthalten die Isomere der 1, 2-; Cyclohexandicarbonsäureester.

Im erfindungsgemäßen Verfahren können folgende aromatische Carbonsäuren eingesetzt werden:
Phthalsäure (Benzol-1,2-dicarbonsäure). Weiterhin können Säuren eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.
Es ist möglich, Alkyl-, Cycloalkyl- sowie Alkoxyalkylester der oben genannten Säuren zu verwenden, wobei diese Reste unabhängig von einander 8 bis 13 C-Atome, insbesondere 9 C-Atome, umfassen. Diese Reste können linear oder verzweigt sein. Hat ein Einsatzprodukt mehr als eine Estergruppe, dann können diese Reste gleich oder verschieden sein.
Im erfindungsgemäßen Verfahren können als Derivat einer aromatischen Polycarbonsäure beispielsweise folgende Verbindungen eingesetzt werden:
Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester.

Es können auch Gemische aus zwei oder mehreren Polycarbonsäureestern eingesetzt werden. Solche Gemische können beispielsweise auf folgenden Wegen erhalten werden:
a) eine Polycarbonsäure wird mit einem Alkohol derart partiell verestert, dass Voll- und Partialester nebeneinander vorliegen.
b) Ein Gemisch von mindestens zwei Polycarbonsäuren wird mit einem Alkohol verestert , wobei ein Gemisch von mindestens zwei Vollestern entsteht.
c) Eine Polycarbonsäure wird mit einem Alkoholgemisch versetzt, wobei ein Gemisch vieler Vollester entstehen kann.
d) Weiterhin kann eine Polycarbonsäure mit einem Alkoholgemisch partiell verestert werden.
e) Weiterhin kann ein Gemisch von mindestens zwei Carbonsäuren mit einem Alkoholgmisch partiell verestert werden.
f) Weiterhin kann ein Gemisch aus mindestens zwei Polycarbonsäuren mit einem Alkoholgemisch partiell verestert werden.

Bei den Umsetzungen a) bis f) können anstelle der Polycarbonsäuren auch deren Anhydride eingesetzt werden.

Großtechnisch werden aromatische Ester, insbesondere die Vollester auf Weg c) häufig aus Alkoholgemischen hergestellt.
Entsprechende Alkoholgemische sind beispielsweise:
C₈-Alkoholgemische, wie 2-Ethylhexanol (2 Isomere), hergestellt durch Aldolkondensation von n-Butyraldehyd und anschließender Hydrierung;
C₉-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C4-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher ein Vielzahl von technischen C₉-Alkoholgemischen.
C₁₀-Alkoholgemische, hergestellt aus Tripropylen durch Hydroformylierung und anschließender Hydrierung; 2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung;
C₁₃-Alkolgemische, hergestellt aus Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer- Tropsch-Synthesen, bei Dehydrierungen von Kohlenwasserstoffen, Metathesereaktionen, beim Polygasverfahren oder anderen technischen Prozessen anfallen.
Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Polycarbonsäuren und den obengenannten Alkoholgemischen, eingesetzt werden. Erfindungsgemäß werden bevorzugt Ester, hergestellt aus Phthalsäure oder Phthalsäureanhydrid und einem Gemisch isomerer Alkohole mit 8 bis 13 C-Atomen, eingesetzt.

Beispiele für technische Phthalate, die im erfindungsgemäßen Verfahren eingesetzt werden können, sind folgende Produkte mit den Handelsnamen:
Jayflex DINP (CAS Nr.68515-48-0); Jayflex DIDP (CAS Nr.68515-49-1); Palatinol 9-P (68515-45-7), Vestinol 9 (CAS Nr. 28553-12-0); Jayflex DIOP (CAS Nr. 27554-26-3); Jayflex UDP (CAS Nr. 68515-47-9); Jayflex DIUP (CAS Nr. 85507-79-5); Jayflex DTDP (CAS Nr.68515-47-9); Jayflex L9P (CAS Nr. 68515-45-7); Jayflex L911P (CAS Nr. 68515-43-5); Jayflex L11P (CAS Nr. 3648-20-2); Witamol 110 (CAS Nr. 68515-51-5); Witamol 118 (Di-n-C8-C10-alkylphthalat) (CAS Nr.71662-46-9); Linplast 812 HP (CAS Nr. 70693-30-0); Palatinol 911 (CAS Nr. 68515-43-5); Palatinol 11 (CAS Nr. 3648-20-2); Palatinol 10 P (CAS Nr. 53306-54-0).

Im Folgenden wird auf die mögliche Stereoisomere des alicyclischen Systems Bezug genommen, wobei zwischen der trans- und cis-Form unterschieden wird. Beispielsweise sind bei der 1,2-Cyclohexandicarbonsäureester, wie bereits erwähnt, die trans-Formen diejenigen Verbindungen, bei denen die Estergruppen entweder axial-axial (a, a) oder äquatorial-äquatorial (e, e) ausgerichtet sind; bei der cis-Verbindung ist eine Estergruppe axial (a) und die andere äquatorial (e) ausgerichtet. Für andere alicyclische Polycarbonsäuren können, wie bereits ausgeführt, bei der Unterscheidung dieser beiden Formen andere Ausrichtungen gelten.

Die erfindungsgemäß hergestellten Gemische enthalten zu über 10 Mol-%, bezogen auf die gesamte Estermenge, die trans-Verbindung(en). Bevorzugt enthalten die Gemische mehr als 15, besonders bevorzugt über 20, ganz besonders bevorzugt über 25 Mol-% des trans-Isomers.

In besonderen Varianten des erfindungsgemäßen Verfahrens werden Phthalsäuredinonylester oder ein Gemisch aus isomeren Phthalsäuredinonylestern zu einem isomeren Gemisch von 1,2-Cyclohexandicarbonsäuredinonylestem, mit einem Anteil des bezüglich der Stellung der Carboxylgruppen am Cyclohexanring in trans-Stellung vorliegenden Isomers von über 10 Mol-% hydriert.
Analog kann ebenso Phthalsäuredi(2-ethylhexyl)ester zu 1,2-Cyclohexandicarbonsäuredi(2-ethylhexyl)ester oder Phthalsäuredidecylester zu 1,2-Cyclohexandicarbonsäuredidecylester umgesetzt werden. Bezüglich der cis-/trans-Isomeren gilt das für den Isononylester Ausgeführte.

Für alle genannten Verbindungsklassen kann der Anteil der trans-Isomere im erhaltenen Gemisch über 15, bevorzugt über 20 Mol-%, ganz besonders bevorzugt über 25 Mol-% liegen.

Beispielsweise liegt bei der erfindungsgemäßen Hydrierung von Vestinol 9 (Phthalsäurediisononylester der Oxeno GmbH) der Gehalt an trans-Verbindungen im Produkt zwischen 11 und 26 Massen-%, wie durch ¹H-NR4R-Spektoskopie bestimmt werden kann.

"Isomere" Phthalsäureester bezeichnen die Strukturisomere, die sich durch die unterschiedlichen am Sauerstoffatom der Estergruppe gebundenen Reste ergeben. Beispielsweise können isomere Nonylphthalate n-Nonylreste sowie verschiedene einfach oder mehrfach verzweigte Reste mit 9 C-Atomen enthalten.

Die erfindungsgemäß hergestellten alicyclischen Polycarbonsäure-estergemische mit hohem trans-Anteil können als Weichmacher in Kunststoffen verwendet verden. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

### Als Vertreter der obigen Gruppen seien beispielsweise folgende Kunststoffe genannt:

Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Butylacrylat-Copolymere, Methylmethacrylat-Butylmethacrylat-Copolymere, Ethylen-VinylacetatCopolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere.
Darüber hinaus können die erfindungsgemäße hergestellten alicyclischen Polycarbonsäureester bzw. das Gemisch zur Modifizierung von Kunststoffmischungen, beispielsweise der Mischung eines Polyolefins mit einem Polyamid, eingesetzt werden.

Geeignete Kunststoffe sind die bereits genannten Verbindungen. Solche Gemische enthalten bevorzugt mindestens 5 Gew.-%, besonders bevorzugt 20-80 Gew.-%, ganz besonders bevorzugt 30-70 Gew.-% der alicyclischen Polycarbonsäureester.

Gemische aus Kunststoffen, insbesondere PVC, die erfindungsgemäß hergestellte alicyclische Polycarbonsäureestergemische enthalten, können beispielsweise in folgenden Produkten enthalten sein:
Gehäuse für Elektrogeräte, wie beispielsweise Küchengeräte, Computergehäuse, Gehäuse und Bauteile von Phono- und Fernsehgeräte, Rohrleitungen, Apparaten, Kabeln, Drahtummantelungen, Isolierbändern, Fensterprofilen, im Innenausbau, im Fahrzeug- und Möbelbau, Plastisolen, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, Fasern für Gewebe.

Weiterhin können Gemische aus Kunststoff, insbesondere PVC, die erfindungsgemäß hergestellte alicyclische Polycarbonsäureestergemische enthalten, beispielsweise zur Herstellung folgender Erzeugnisse verwendet werden:
Ein Gehäuse für Elektrogeräte, eine Rohrleitung, eine Vorrichtung, ein Kabel, eine Draht-Ummantelung, ein Fensterprofil, ein Bodenbelag, ein medizinischer Artikel, ein Spielzeug, eine Lebensmittelverpack-ung, eine Dichtung, eine Folie, eine Verbundfolie, eine Schallplatte, Kunstleder, ein Verpackungsbehälter, eine Klebebandfolie, Bekleidung, eine Beschichtung, oder eine Faser für Gewebe.
Neben den obengenannten Anwendungen können die erfindungsgemäße hergestellten alicyclischen Polycarbonsäureestergemische als Schmierölkomponente, als Bestandteil von Kühlflüssigkeiten und Metallbearbeitungsflüssigkeiten verwendet werden.

Die folgenden Beispiele sollen die Erfindung erläutern, ohne den Schutzbereich, definiert durch die Patentansprüche, einzuschränken.

### Analytik:

Das Verhältnis von cis- und trans-1.2-Cyclohexancarbonsäurediester wurde durch ¹H-NMR-Spek-toskopie bestimmt.
- Messgerät:: NMR-Spektrometer Avance DPX-360 der Firma Bruker
- Messfrequenz:: 360 MHz
- Probenkopf:: QNP-Probenkopf, 5mm
- Lösungsmittel:: CDCl₃ (Deuterierungsgrad 99,8 %)
- Standard:: Tetramethylsilan (TMS)
- Messtemperatur:: 303 K
- Anzahl Scans:: 32
- Delay:: 1 s
- Aquisitionszeit:: 4,4 s
- Spektrale Breite:: 7440,5 Hz
- Pulswinkel:: 30°
- Pulslänge:: 3,2µs

Zur Aufnahme der ¹H-NMR-Spektren wurden z. B. ca. 20 mg der Probe in ca. 0,6 ml CDCl₃ (mit 1 Gew.-% TMS) gelöst. Die Spektren wurden unter den oben angegebenen Bedingungen aufgenommen und auf TMS = 0 ppm referenziert.

In den erhaltenen ¹H-NMR-Spektren ließen sich die Methinsignale der cis- und trans-Dialkylhexahydrophthalate mit den chemischen Verschiebungen von ca. 2,8 ppm bzw. 2,6 ppm unterscheiden. Dabei entsprach das zu tieferem Feld verschobene Signal (größerer ppm-Wert) der cis-Verbindung. Zur Quantifizierung der Isomere wurden die Integrale von 3,0 ppm bis 2,7(2) ppm und von 2,7(2) ppm bis 2,5 ppm bestimmt, wobei die Trennung der beiden Integrale in der Mittel zwischen den Signalen erfolgte. Aus den Intensitätsverhältnissen konnte das Verhältnis der beiden isomeren Strukturen bestimmt werden.
Eingesetzt wurde der handelsübliche Katalysator H 10126 rs, hergestellt von der Degussa AG. Seine Eigenschaften sind durch den Hersteller wie folgt beschrieben:

| | |
|---|---|
| w (Ni) : | ca. 32 % |
| w (ZnO) : | ca 30 % |
| w (SiO₂) : | ca. 24 % |

| Form : | Tabletten |
|---|---|
| Schüttdichte : | ca. 1,00 g/cm³ |
| spez. Porenvolumen : | ca. 0,4 cm³/g |
| BET-Oberfläche : | ca 160 m²/g |

### Aktivierungsvorschrift zur Reduktion der oxidischen Form von H 10126

Das Katalysatorbett wurde im Stickstoffstrom bis auf 250 °C aufgeheizt. Nachdem der O₂-Gehalt im Ausgangsgas auf unter 0,2 % gesunken war, wurde langsam Wasserstoff bis zu einer Eingangskonzentration von 5 Vol.-% eingespeist. Die Temperatur in der Katalysatorschüttung wurde dann um ca. 25 °C/h bis auf 350 °C erhöht. Nachdem die bei der Reduktion auftretende Wasserbildung nachließ, wurde die Wasserstoffkonzentration allmählich auf 100 % angehoben. Die Temperatur von 300 - 350 °C wurde noch 36 Stunden lang im Wasserstoffstrom aufrechterhalten. Während der Hauptreduktionsphase betrug das Wasserstoffatom 500 Nl/1/h. Die Aktivierung wurde bei Normaldruck durchgeführt.

### Beispiel 1

82 g des Ni / Zn-Katalysators H 10126 wurden in einem Katalysatorkörbchen vorgelegt, in einem 600 ml-Druckreaktor sorgfältig nach Herstellerangabe im Wasserstoffstrom reduziert und dann mit 590 g flüssigem Diisononylphthalat **(Vestinol 9)** versetzt. Die Hydrierung des DINP erfolgte mit reinem Wasserstoff bei einem Druck von 200 bar und einer Temperatur von 120° C. Nach Hydrierung des Einsatzstoffes wurde der Reaktor entspannt und das Reaktionsgemisch analysiert. Der Umsatz an DINP betrug danach 99,9 %: Die Ausbeute an Cyclohexan-I,2-dicarbonsäuredi(isononyl)-ester (DINCH) lag bei 99,8 %, wobei der Anteil der cis-Verbindungen bei 84 % lag (bestimmt über ¹H-NMR).
Analog Beispiel 1 wurden weitere Hydrierversuche mit dem gleichen Katalysator durchgeführt. Es wurden Drücke und Temperaturen variiert. Als Edukte wurden Diisononylphthalat (Vestinol 9) und Di(2-ethylhexyl)phthalat (DOP) eingesetzt. Die Versuche wurden in der folgenden Tabelle zusammengefasst.

| **Beispiel** | **Edukt** | **Druck (bar)** | **Temperatur (°C)** | **Ausbeute (%)** | **cis-Anteil (%)** |
|---|---|---|---|---|---|
| 1 | Vestinol9 | 200 | 120 | >99,8 | 84 |
| 2 | Vestinol9 | 200 | 200 | >99,9 | 78 |
| 3 | Vestinol9 | 50 | 200 | 99,8 | 74 |
| 4 | DOP | 50 | 200 | >99,9 | 71 |

## Patentansprüche

1. Verfahren zur Herstellung von alicyclischen Polycarbonsäureestergemischen umfassend die Isomeren der 1,2-Cyclohexandicarbonsäureester, enthaltend mindestens zwei Isomere bezüglich der Stellung der Estergruppen am Ringsystem, wobei die Alkhoholkomponenten der Polycarbonsäureestergemische Alkoxyalkyl-, Cycloalkyl-, und/oder Alkylgruppen mit 8 bis 13 Kohlenstoffatomen, verzweigt oder unverzweigt, jeweils gleich oder unterschiedlich sind und der Anteil der trans-Isomere über 10 Mol-% beträgt, **dadurch gekennzeichnet, dass** das alicyclische Polycarbonsäureestergemisch durch Hydrierung der entsprechenden aromatischen Polycarbonsäureester hergestellt wird,
und die Hydrierung der aromatischen Polycarbonsäureester an einem Katalysator, der mindestens ein Metall aus der Triade Eisen, Kobalt, Nickel zusammen mit mindestens einem Metall der II, III, IV, V und/oder VI Nebengruppe des Periodensystems enthält, durchgeführt wird.

2. Verfahren nach Anspruch 1,
**dadurch gekennzeichnet,**
**dass** Phthalsäuredinonylester oder ein Gemisch aus isomeren Phthalsäuredinonylestern zu einem isomeren Gemisch von Cyclohexandicarbonsäuredinonylester hydriert wird.

3. Verfahren nach Anspruch 1,
**dadurch gekennzeichnet,**
**dass** Phthalsäuredecylester oder ein Gemisch aus isomeren Phthalsäuredecylestern zu einem isomeren Gemisch von Cyclohexandicarbonsäuredidecylestern hydriert wird.

4. Verfahren nach Anspruch 1,
**dadurch gekennzeichnet,**
**dass** Phthalsäuredi(octyl)ester oder ein Gemisch aus isomeren Phthalsäuredi(octyl)ester zu einem isomeren Gemisch von Cyclohexandicarbonsäuredi(octyl)ester hydriert wird.

## Claims

1. Process for preparing alicyclic polycarboxylic ester mixtures encompassing the isomers of the cyclohexane-1,2-dicarboxylic esters, comprising at least two isomers with respect to the position of the ester groups on the ring system, where the alcohol components of the polycarboxylic ester mixtures are alkoxyalkyl, cycloalkyl, and/or alkyl groups having from 8 to 13 carbon atoms, branched or unbranched, and in each instance identical or different and the proportion of the trans isomers is above 10 mol%, **characterized in that** the alicyclic polycarboxylic ester mixture is prepared by hydrogenating the corresponding aromatic polycarboxylic esters, and the hydrogenation of the aromatic polycarboxylic esters is carried out on a catalyst which comprises at least one metal from the triad iron, cobalt, nickel, together with at least one metal of the IInd, IIIrd, IVth, Vth, and/or VIth transition group of the Periodic Table.

2. Process according to Claim 1,
**characterized in that**
dinonyl phthalate or a mixture of isomeric dinonyl phthalates is hydrogenated to give an isomeric mixture of dinonyl cyclohexanedicarboxylates.

3. Process according to Claim 1,
**characterized in that**
decyl phthalate or a mixture of isomeric decyl phthalates is hydrogenated to give an isomeric mixture of didecyl cyclohexanedicarboxylates.

4. Process according to Claim 1,
**characterized in that**
di(octyl) phthalate or a mixture of isomeric di(octyl) phthalates is hydrogenated to give an isomeric mixture of di(octyl) cyclohexanedicarboxylates.

## Revendications

1. Procédé de préparation de mélanges alicycliques d'esters d'acide polycarboxylique comprenant les isomères de l'ester d'acide 1,2-cyclohexanedicarboxylique, contenant au moins deux isomères, selon la position des groupes ester au niveau du système cyclique, les composants alcooliques des mélanges d'esters d'acide polycarboxylique étant des groupes alcoxyalkyle, cycloalkyle et/ou alkyle ayant 8 à 13 atomes de carbone, ramifiés ou non ramifiés, respectivement identiques ou différents et
la part des isomères trans étant supérieure à 10 % en moles,
**caractérisé en ce que**
le mélange alicyclique d'esters d'acide polycarboxylique est préparé grâce à une hydrogénation des esters d'acide polycarboxylique aromatiques correspondants,
et l'hydrogénation des esters aromatiques d'acide polycarboxylique est mise en oeuvre sur un catalyseur qui contient au moins un métal issu de la triade fer, cobalt, nickel en commun avec au moins un métal des sous-groupes II, III, IV, V et/ou VI du système périodique.

2. Procédé selon la revendication 1,
**caractérisé en ce que**
l'ester dinonylique de l'acide phtalique ou un mélange d'esters dinonyliques d'acide phtalique isomères est hydrogéné pour donner un mélange isomère d'esters dinonyliques d'acide cyclohexanedicarboxylique.

3. Procédé selon la revendication 1,
**caractérisé en ce que**
l'ester décylique de l'acide phtalique ou un mélange d'esters décyliques d'acide phtalique isomères est hydrogéné pour donner un mélange isomère d'esters didécyliques d'acide cyclohexanedicarboxylique.

4. Procédé selon la revendication 1,
**caractérisé en ce que**
l'ester di(octylique) de l'acide phtalique ou un mélange d'esters di(octyliques) d'acide phtalique isomères est hydrogéné pour donner un mélange isomère d'esters di(octylique) d'acide cyclohexanedicarboxylique.
